# EUROPEAN PATENT SPECIFICATION

(11) **EP 0 623 592 B1**
(45) Date of publication and mention of the grant of the patent: **27.06.2001**
(21) Application number: 94201161.0
(22) Date of filing: 27.04.1994
(51) Int. Cl.: C07C 271/22, A61K 31/27, C07D 295/30, C07D 295/088, C07C 311/16, C07D 333/38, C07D 307/38, C07K 5/06, C07K 5/08, A61K 31/18

(54) **Peptide analogs as irreversible interleukin-1beta protease inhibitors**
Peptide-Analoge mit irreversibler Interleukin-1 Beta-Protease Inhibitoren
Analogues de peptides à adivité inhibitrice de l'interleukin 1 bêta protéase

(30) Priority: 29.04.1993 US 55051
(43) Date of publication of application: 09.11.1994
(73) Proprietor: VERTEX PHARMACEUTICALS INCORPORATED, Cambridge, MA 02139-4242 (US)
(72) Inventor: Dolle, Roland E., c/o Sterling Winthrop Inc., New York, New York 10016 (US); Osifo, Irennegbe K., c/o Sterling Winthrop Inc., New York, New York 10016 (US); Schmidt, Stanley J., c/o Sterling Winthrop Inc., New York, New York 10016 (US); Hoyer, Denton W., c/o Sterling Winthrop Inc., New York, New York 10016 (US); Ross, Tina M., c/o Sterling Winthrop Inc., New York, New York 10016 (US); Chaturvedula, Prasad, c/o Sterling Winthrop Inc., New York, New York 10016 (US); Prouty, Catherine P., c/o Sterling Winthrop Inc., New York, New York 10016 (US); Awad, Mohamed M. A., c/o Sterling Winthrop Inc., New York, New York 10016 (US); Salvino, Joseph M., c/o Sterling Winthrop Inc., New York, New York 10016 (US); Rinker, James M., c/o Sterling Winthrop Inc., New York, New York 10016 (US); Lodge, Eric P., c/o Sterling Winthrop Inc., New York, New York 10016 (US); Singh, Jasbir, c/o Sterling Winthrop Inc., New York, New York 10016 (US); Ator, Mark A., c/o Sterling Winthrop Inc., New York, New York 10016 (US)
(74) Representative: Le Guen, Gérard

(56) References cited:
- EP-A- 0 272 671
- WO-A-91/15577
- WO-A-93/09135
- WO-A-93/16710
- NATURE vol. 356 , 30 April 1992 , LONDON GB pages 768 - 774 N. A. THORNBERRY ET AL. 'A novel heterodimeric cysteine protease is required for interleukin-1beta processing in monocytes'
- BIOCHEMISTRY vol. 30, no. 19 , 1991 , EASTON, PA US pages 4678 - 4687 A. KRANTZ ET AL. 'Peptidyl (acyloxy)methyl ketones and the quiescent affinity label concept: the departing group as a variable structural element in the design of inactivators of cysteine proteinases'
- JOURNAL OF BIOLOGICAL CHEMISTRY vol. 265, no. 24 , 1990 , BALTIMORE, MD US pages 14526 - 14528 P. R. SLEATH ET AL. 'Substrate specificity of the protease that processes human interleukin-1beta'
- BIOCHEMISTRY vol. 33, no. 13 , 5 April 1994 , EASTON, PA US pages 3934 - 3940 N. A. THONBERRY ET AL. 'Inactivation of interleukin-1beta converting enzyme by peptide (acyloxy)methyl ketones'
- JOURNAL OF MEDICINAL CHEMISTRY vol. 37, no. 5 , 1994 , WASHINGTON US pages 563 - 564 R. E. DOLLE ET AL. 'P1 aspartate-based peptide alpha-((2,6,dichlorobenzoyl)oxy)methyl ketones as potent time-dependant inhibitors of interleukin-1beta-converting enzyme'

## Description

This invention relates to peptide analogs that are interleukin-1β protease inhibitors. More particularly, the invention provides α-substituted methyl ketones derived from aspartic acid and the closed hemi-ketal forms thereof as inhibitors of interleukin 1-β protease.

Enzymes involved in the catalytic degradation of proteins by hydrolyzing peptide bonds are known as proteases or proteinases. Proteinases are believed to be involved in various disease states including inflammation, metastasis, tissue damage, bone resorption and muscle degeneration in dystrophic diseases. Proteinases are divided into classes according to their catalytic mechanisms, such as serine-, cystein-, aspartic- and metallo-proteinases. For each class of proteinases, the catalytic site of the enzyme lies in the cleft on the surface of the enzymes in which reside the specificity subsites that bind amino acid side chains and the polypeptide backbone. In designing proteinase inhibitors, it is important to optimize the subsite binding characteristics with appropriate amino acid substrate analogs.

This invention relates to peptide substrates modified with affinity labels that inhibit interleukin-1β protease (hereinafter IL-1β protease). These inhibitors are thought to act by alkylating the cysteine sulfhydryl group (cys 285) within the catalytic site of IL-1β protease. Affinity labelling has been used since the 1960's to prepare irreversible peptide-based inhibitors which act to alkylate the active sites of cysteine proteases. A variety of affinity labels and amino acid sequences have been synthesized to improve the binding of these modified peptide inhibitors to the enzyme's active site. These affinity labels include peptidyl halomethyl ketones, peptidyl diazomethyl ketones, epoxysuccinyl peptides and peptidyl methylsulphonium salts as reviewed by D. Rich in Chapter 4 of "Proteinase Inhibitors", Barret, A.J. and Salvesen, G., eds., Elsevier, 1986. More recently, peptide acyloxymethyl and aryloxymethyl ketones have also been described as affinity lables (Krantz, A. et al., Biochemisty, 30, p. 4678-4687, 1991). Current research (see for example European Patent Application, Pub. No. 01 5,748 A2; PCT International Publication No. WO 91/15577; Chapman, K.T., Biorganic & Medicinal Chem. Lett. 1992, 2, 613-618) has been directed towards understanding the enzyme binding specificity requirements in designing novel small molecular weight protease inhibitors that are efficacious, safe and have specificity for IL-1β protease which is believed to play an important role in many disease states (see Epstein, F.H., New Engl. Jrl. of Med., 328, p. 106-113, 1993).

WO 91/15577 discloses inhibitors of interleukin-1β-protease of the formula Z-Q₂-Asp-Q₁ in which Z is an amino protecting group, Q₂ is 0 to 4 aminoacids such that the sequence Q₂-Asp substantially corresponds to at least a portion of the sequence Ala-Tyr-Val-His-Asp and Q₁ is an electronegative leaving group.

None of the compounds proposed as examples comprises cyclic α-aminoacid. Moreover, the whole disclosure of this document does not suggest the incorporation of such aminoacids.

Biochemistry 30(19), 1991, 4678-4687 describes potent inhibitors of cathepsin B which is a cysteine protease. Said inhibitors are (acyloxy)methylketones of the formula Z-[AA₂]-[AA₁]-CH₂-O-CO-Ar. However both AA₁ and AA₂ are non cyclic aminoacid.

Disease states in which IL-1β protease inhibitors may be useful as therapeutic agents include: infectious diseases, such as meningitis and salpingitis; septic shock, respiratory diseases; inflammatory conditions, such as arthritis, cholangitis, colitis, encephalitis, endocerolitis, hepatitis, pancreatitis and reperfusion injury, immune-based diseases, such as hypersensitivity; auto-immune diseases, such as multiple sclerosis; bone diseases; and certain tumors.

it is an object of the present invention to provide novel peptidyl substrate analogs modified with electronegative leaving groups that bind at the active site of IL-1β protease and inhibit IL-1β protease activity. IL-1β protease cleaves a biologically inactive 34kD precursor of IL-1β to form the biologically active 17kD cytokine. This cleavage occurs at the peptidyl sequence of Val-His-Asp/-Ala-Pro-Val.

It is another object of the present invention to provide compositions comprising the above-referred to compounds.

It is a further object of the present invention to provide a method of use of the composition for the treatment of the above-identified disease states.

According to the present invention, there is provided a compound of the formula (I) and a pharmaceutically acceptable salt thereof: 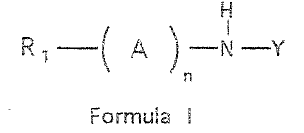 wherein
- n =: 1 or 2 ;
- Y =: 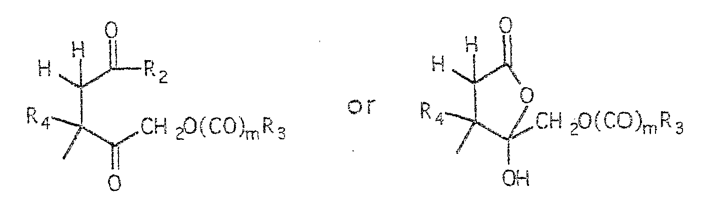
- m =: 0,1;
- R₃ =: a singularly or multiply substituted aryl selected from phenyl and naphthyl ring wherein the substituents are independently selected from the group consisting of:
(1) H;
(2) halogen;
(3) OH
(4) CF₃;
(5) NO₂;
(6) OR₅
(7) COR₉;
(8) NR₆COR₁₀;
(9) CONR₅R₆
(10) SO₂NR₅R₆;
(11) SO₂R₆;
(12) COOR₁₁;
(13) 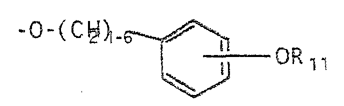 and
(14) lower alkyl and lower cycloalkyl;
- R₅ =: (1) lower straight chain or branched alkyl, lower cycloalkyl
(2) (CR₆R₇)₀₋₆-aryl;
(3) (CR₆R₇)₀₋₆-heteroaryl or
(4) (CR₆R₇)₂₋₆-R₈;
- R₆ and R₇: are independently H, lower straight chain or branched alkyl, benzyl, cycloalkyl or aryl wherein aryl is defined as above and heteroaryl includes pyridyl, thienyl, furyl, thiazolyl, imidazolyl, pyrazolyl, isoxazolyl, benzimidazolyl, pyrazinyl, pyrimidyl, quinolyl, isoquinolyl, isothiazolyl, benzofuranyl, isoxazolyl, triazinyl and tetrazolyl;
- R₈ =: (1) OCH₂CH₂OR₆;
(2) OCH₂CH₂NR₆R₇
(3) NR₆CH₂CO₂R₆;
(4) 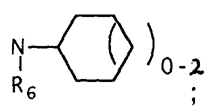
(5) 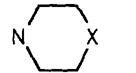 X = O,S and
(6) NR₆R₇ wherein R₆ and R₇ are as above defined;
- R₉ =: (1) lower straight chain or branched alkyl, lower cycloalkyl
(2) (CR₆R₇)₀₋₆-aryl;
(3) (CR₆R₇)₀₋₆-heteroaryl; or
(4) (CR₆R₇)₀₋₆-R₈, wherein R₆, R₇ and R₈ are as above defined;
- R₁₀ =: (1) R₉;
(2) OR₁₁; or
(3) NR₆R₁₁,
wherein
- R₁₁ =: (1) lower straight chain or branched alkyl, lower cycloalkyl
(2) (CR₆R₇)₁₋₆-aryl;
(3) (CR₆R₇)₁₋₆-heteroaryl; or
(4) (CR₆R₇)₂₋₆-R₈, and R₆, R₇, R₈ and R₉ are as above defined;
- R₄ =: H or deuterium;
- R₂ =: (1) OR_{6;}
(2) NR₆OR₇ or
(3) NR₆R_{7,} and R₆ and R₇ are as above-defined;
- A=: an amino acid selected from the group consisting of 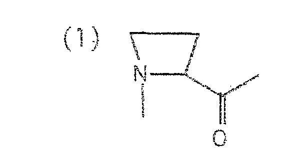 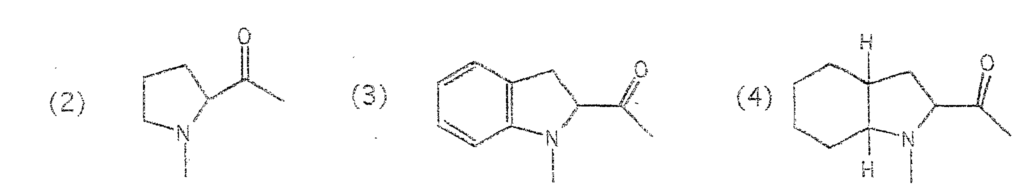 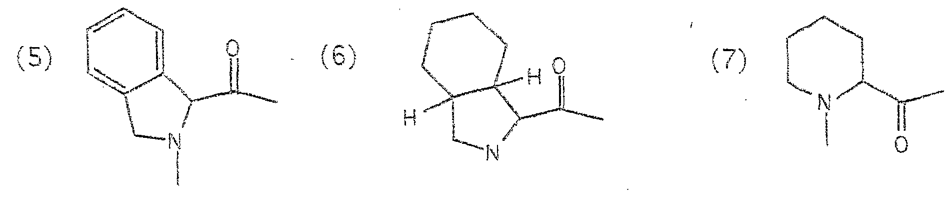 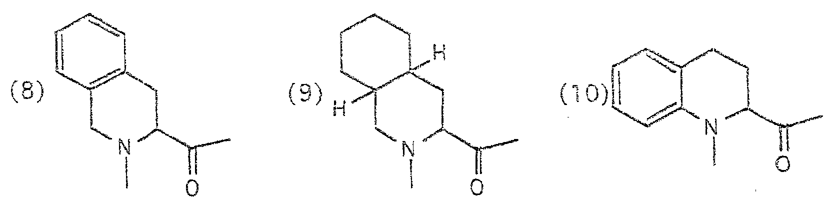 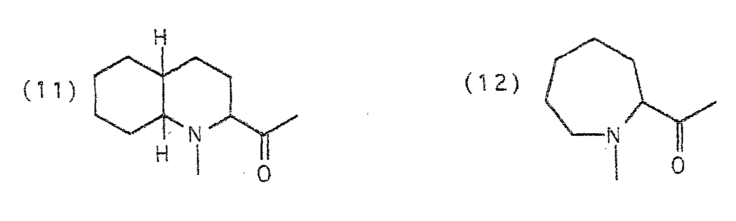 and 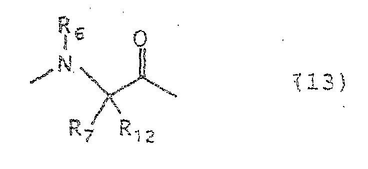 wherein R₆ and R₇ are as defined above; and
- R₁₂: is independently
(1) H ; or
(2) (CR₆R₇)₁₋₆-R₁₃,
wherein R₆ and R₇ are as above-defined;
- R₁₃ =: (1) H;
(2) F;
(3) CF₃
(4) OH;
(5) OR₁₁;
(6) NR₆R₁₄
(7) cycloalkyl;
(8) aryl
(9) heteroaryl;
(10) SH
(11) SR₁₁;
(12) CONR₅R₆
(13) COOR₅ or
(14) 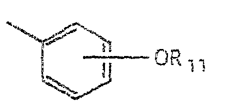 wherein R₅, R₆, and R₁₁ are as defined above ;
- R₁₄ =: (1) R₇;
(2) COR₁₀;
(3) SO₂NR₅R₆ or
(4) 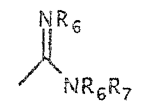 wherein R₅, R₆, R₇ and R₁₀ are as defined above;
- R₁ is: an acyl group of the formula (III) 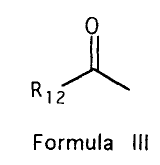 wherein
- R₁₂: is
(1) OR_{5;}
(2) NR₅R_{6;}
(3) R_{5;}
(4) -CH=CHR₅
(5) 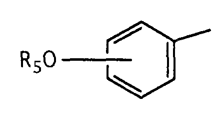
(6) 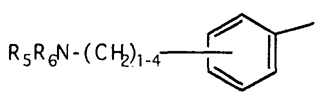
(7) 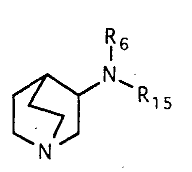 or
(8) 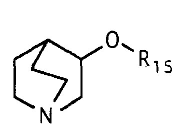 wherein
- R₁₅ =: single bond, (CH₂)₂₋₆-NR₆-, (CH₂)₂₋₆-O- and R₅ and R₆ are as above defined; or a sulfonyl group of the formula (IV) 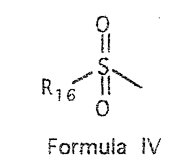 wherein
- R₁₆: is
(1) R₅
(2) 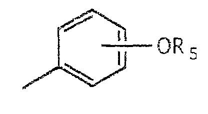 or
(3) 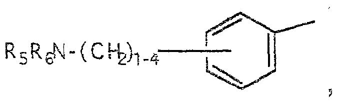 wherein R₅ and R₆ are as above-defined.
the compounds in which all A groups represent a radical of formula (13) being excluded,
with the proviso that when n=1, R₃ does not represent phenyl optionally substituted by one to three radicals selected from lower alkyl, lower alkoxy, trifluoromethyl, halogen, nitro, carbamoyl and (C₁-C₆)alkoxycarbonyl;
it beeing understood that lower alkyl designates an alkyl group having 1 to 7 carbon atoms and lower cycloalkyl designates a cycloalkyl group having 3 to 6 carbon atoms. As used herein the term pharmaceutically acceptable salts include the acid and base addition salts.

The term acid addition salts refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

The term base addition salts include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts derived from pharmaceutically acceptable organic non-toxic bases including salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, trimethamine, dicyclohexylamine, lysine, arginine, histidine, caffeine, procaines, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, trimethamine, dicyclohexylamine, choline and caffeine.

Alkyl means a saturated or unsaturated aliphatic hydrocarbon which may be either straight- or branched-chain. Preferred groups have no more than about 12 carbon atoms and may be methyl, ethyl and structural isomers of propyl, butyl, pentyl, hexyl, heptyl, octyl, nonyl, decyl, undecyl and dodecyl.

Lower alkyl means an alkyl group as above, having 1 to 7 carbon atoms. Suitable lower alkyl groups are methyl, ethyl, n-propyl, isopropyl, butyl, tert-butyl, n-pentyl, neopentyl, n-hexyl, and n-heptyl.

Substituted phenyl means a phenyl group in which one or more of the hydrogens has been replaced by the the same or different substituents including halo, lower alkyl, nitro, amino, acylamino, hydroxyl, lower alkoxy, aryl, heteroaryl, lower alkoxy, alkylsulfonyl, trifluoromethyl, morpholinoethoxy, morpholinosulfonyl, and carbobenzoxymethyl sulfamoyl.

Halogen means chloride, fluoride, bromide or iodide.

Lower cycloalkyl means cycloalkyl having C₃ to C₆ carbon atoms.

In another aspect of the invention the invention concerns the use of a compound of formula (I), a pharmaceutically acceptable salt thereof or a pharmaceutical composition thereof for the preparation of a medicament for inhibiting interleukin-1β protease activity in a mammal in need of such treatment. IL-1β mediated disease states and disorders which can be thus treated include : infectious diseases, such as meningitis and salpingitis ; septic shock, respiratory diseases ; inflammatory conditions, such as arthritis, cholangitis, colitis, encephalitis, endocerolitis, hepatitis, pancreatitis and reperfusion injury, immune-based diseases, such as hypersensivity ; auto-immune diseases, such as multiple sclerosis ; bone diseases ; and certain tumors.

According to preferred embodiments of the invention the compounds of formula I, their salts or pharmaceutical compositions thereof are used for the preparation of medicaments for :
- treating IL-1β mediated disease : infectious disease, respiratory disease, inflammatory conditions ;
- treating or preventing a disease or disorder selected from meningitis, salpingitis, septic shock, arthritis, cholangitis, colitis, encephalitis, endocerolitis, hepatitis, pancreatitis, reperfusion injury, hypersensivity and multiple slcerosis in a mammal ;
- treating or preventing arthritis, septic shock, colitis or pancreatitis.
in the practice of this invention an effective amount of a compound of the invention or a pharmaceutical composition thereof is administered to the subject in need of, or desiring, such treatment. These compounds or compositions may be administered by any of a variety of routes depending upon the specific end use, including orally, parenterally (including subcutaneous, intraarticular, intramuscular and intravenous administration), rectally, buccally (including sublingually), transdermally or intranasally. The most suitable route in any given case will depend upon the use, the particular active ingredient, and the subject involved. The compound or composition may also be administered by means of controlled-release, depot implant or injectable formulations as described more fully herein.

In general, for the uses as described in the instant invention, it Is expedient to administer the active ingredient in amounts between 0.1 and 100 mg/kg body weight, most preferably from 0.1 to 30 mg/kg body weight for human therapy, the active ingredient being administered preferably in the range of from 0.1 to 20-50 mg/kg/day. This administration may be accomplished by a single administration, by distribution over several applications or by slow release in order to achieve the most effective results. When administered as a single dose, administration will most preferably be in the range of from 0.1 to 10 mg/kg of body weight.

The exact dose and regimen for administration of these compounds and compositions will necessarily be dependent upon the needs of the individual subject being treated, the type of treatment, and the degree of affliction or need. In general, parenteral administration requires lower dosage than other methods of administration which are more dependent upon absorption.

A further aspect of the present invention relates to pharmaceutical compositions comprising as an active ingredient a compound of the present invention of formula (1) in admixture with a pharmaceutically acceptable, non-toxic carrier. As mentioned above, such compositions may be prepared for use for parenteral (subcutaneous, intraarticular, intramuscular or intravenous) administration, particularly in the form of liquid solutions or suspensions; for oral, rectal or buccal administration, particularly in the form of tablets or capsules; transdermally; or intranasally, particularly in the form of powders, nasal drops or aerosols.

When administered orally (or rectally) the compounds will usually be formulated into a unit dosage form such as a tablet , capsule, suppository or cachet. Such formulations typically include a solid, semi-solid or liquid carrier or diluent. Exemplary diluents and vehicles are lactose, dextrose, sucrose, sorbitol, mannitol, starches, gum acacia, calcium phosphate, mineral oil, cocoa butter, oil of theobroma, alginates, tragacanth, gelatin, syrup, methylcellulose, polyoxyethylene sorbitar monolaurate, methyl hydroxybenzoate, propyl hydroxybenzoate, talc, and magnesium stearate.

The compositions may be prepared by any of the methods well-known in the pharmaceutical art, for example as described in Remington's Pharmaceutical Sciences, 17th edition, Mack Publishing Company, Easton, PA, 1985. Formulations for parenteral administration may contain as common excipients sterile water or saline, alkylene glycols such as propylene glycol, polyalkylene glycols such as polyethylene glycol, oils of vegetable origin, hydrogenated naphthalenes and the like. Examples of vehicles for parenteral administration include water, aqueous vehicles such as saline, Ringer's solution, dextrose solution, and Hank's solution and nonaqueous vehicles such as fixed oils (such as corn, cottonseed, peanut, and sesame), ethyl oleate, and isopropyl myristate. Sterile saline is a preferred vehicle and the compounds are sufficiently water soluble to be made up as a solution for all foreseeable needs. The vehicle may contain minor amounts of additives such as substances that enhance solubility, isotonicity, and chemical stability, for example, antioxidants, buffers, and preservatives. For oral administration, the formula can be enhanced by the addition of bile salts and also by the addition of acylcarnitines (Am. J. Physiol. 251:332 (1986)). Formulations for nasal administration may be solid and contain as excipients, for example, lactose or dextran, or may be aqueous or oily solutions for administration in the form of nasal drops or metered spray. For buccal administration typical excipients include sugars, calcium stearate, magnesium stearate, pregelatinated starch, and the like.

When formulated for nasal administration the absorption across the nasal mucous membrane is enhanced by surfactant acids, such as for example, glycocholic acid, cholic acid, taurocholic acid, desoxycholic acid, chenodesoxycholic acid, dehydrocholic acid, glycodeoxycholic acid, and the like. (See, B.H. Vickery, "LHRH and its Analogs-Contraception and Therapeutic Applications", Pt. 2, B.H. Vickery and J.S. Nester, Eds., MTP Press, Lancaster, UK, 1987).

Compounds of the present invention are prepared using the procedure described generally in Schemes I, II and III and in more detail as described in the Examples, but the scope of the invention is in no way to be construed as limited thereto. 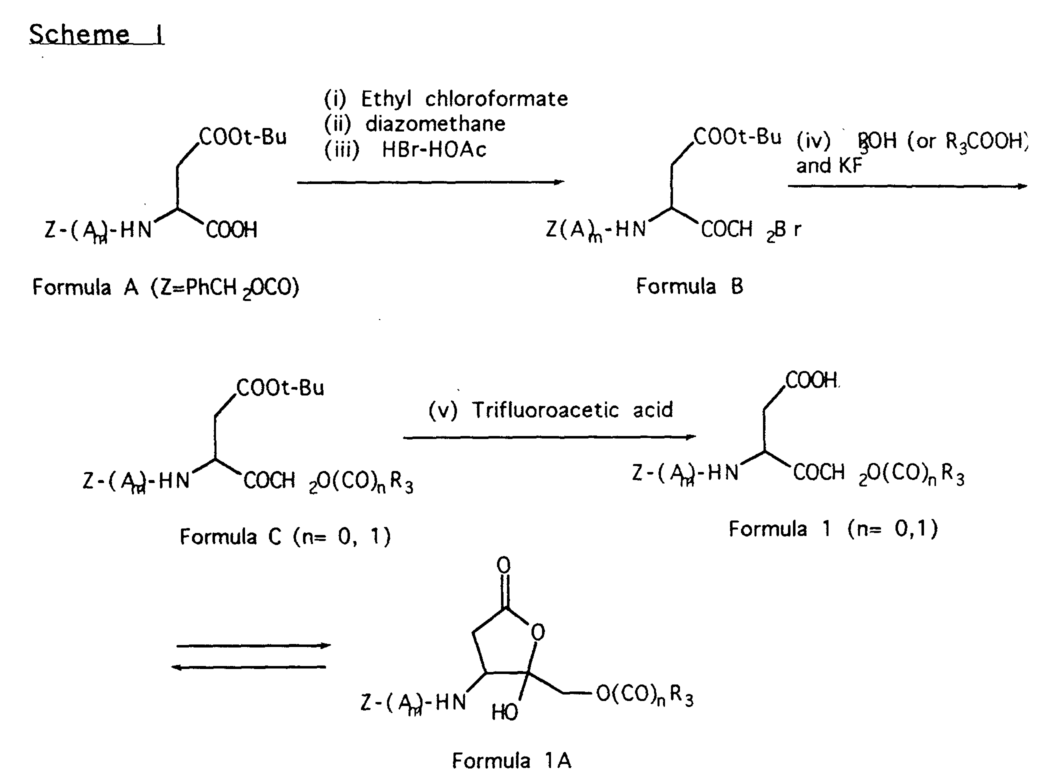 wherein A, R₃ and m are as defined in formula (I) and Z is benzyloxycarbonyl. 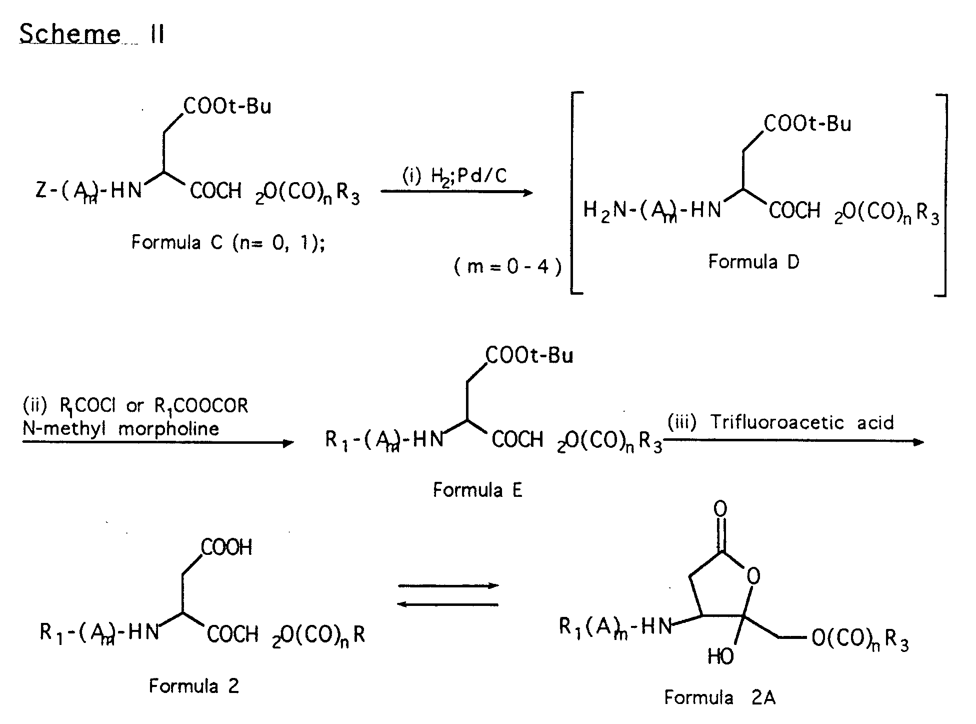 wherein Z, A, R₁, R₃, m and n are as defined in formula (I).

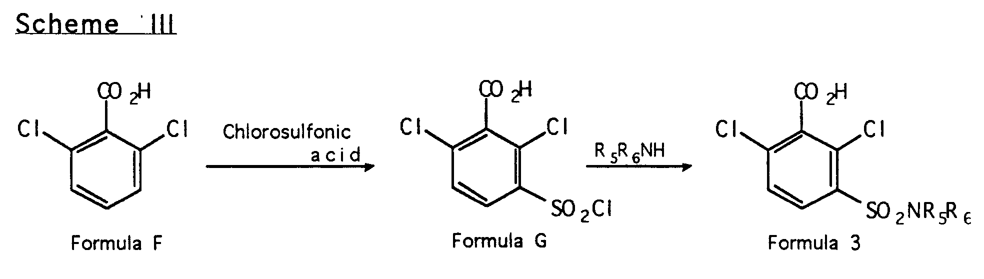

The synthesis of the disclosed interleukin enzyme (ICE) inhibitors was conducted by one of two methods depicted in Schemes I and II. For inhibitors which contained an N-terminal benzyloxycarbonyl group ("Z" group), N-benzyloxycarbonyl-L-aspartic acid mono t-butyl ester or other benzyloxycarbonyl protected aspartic acid-based peptides (Formula A) were used as starting materials. The synthesis of the requisite peptides are readily carried out by a variety of methods known to those practicing in the art of peptide chemistry. The aspartic acid-based peptide (Formula A) is reacted with ethyl chloroformate and N-methyl morpholine in tetrahydrofuran (THF) at low temperature (ca. -15°C) for approximately 30 min. This generates a mixed anhydride in solution thereby activating the free carboxylate toward nucleophilic attack. Other activating reagents (for example isopropyl chloroformate), solvents (diethyl ether, dioxane), and tertiary amine bases (diisopropyl ethylamine, triethylamine) can be used in place of the above preferred reagents to form a reactive carboxylate species. The pre-formed mixed anhydride is treated (without isolation) with a solution of diazomethane in diethyl ether. The diazomethane reagent is prepared under standard conditions from DIAZALD® using a commercially available (Aldrich) diazomethane generator. A one to two molar excess of diazomethane is added and the reaction mixture is warmed from -15°C to 25°C over a 20 min period. During this time, diazomethane reacts with the mixed anhydride to form an α-diazoketone. The α-diazoketone is not isolated and the reaction mixture is treated directly with an excess of a 1:1 solution of 48% hydrobromic (HBr) and glacial acetic (HOAc) acids. The mixture of acids are added dropwise to the α-diazoketone and the reaction mixture is subsequently stirred for at least 15 minutes. This treatment with 1:1 48% HBr and glacial HOAc decomposes the α-diazoketone to yield the desired N-benzyloxycarbonyl-L-aspartic acid mono t-butyl ester α-bromoketone (Formula B) and nitrogen gas as a by-product. The bromomethyl ketone is typically isolated as an oil using standard procedures which are apparent to those skilled in the art. The α-bromoketone so obtained is of sufficient purity to be used in all subsequent reactions. However, the ketone can be further purified by high pressure liquid chromatography (HPLC), if analytically pure material is desired.

The t-butyl ester α-bromoketone (Formula B) is subsequently reacted with a variety of phenols, naphthols, and arylcarboxylic acids. This is conducted by exposing the bromomethyl ketone to an excess of the phenol, naphthol or arylcarboxylic acid in dimethylformamide containing sodium or potassium hydride or potassium fluoride. The reaction can be conveniently monitored by thin layer chromatography (TLC) and once the TLC indicates that displacement of the bromide with the phenol or carboxylate is completed, the product is isolated using standard procedures. The desired aspartic acid mono t-butyl ester α-aryloxymethyl- or α-arylacyloxymethyl ketone (Formula C) may be purified by conventional methods including recrystallization and silica gel column chromatography.

The remaining synthetic transformation to generate the ICE inhibitors is the hydrolysis of the t-butyl ester function. This is conducted by exposing the t-butyl ester (Formula C) to a 25% solution of trifluoroacetic acid (TFA) in methylene chloride at 25°C. The de-esterification is typically complete within 3 hrs. Removal of the volatile TFA and organic solvent affords the aspartic acid (Formula 1). The yield of the reaction is quantitative in most instances, providing the t-butyl ester starting material is of high purity. Purification, if required, can be performed by recrystallization or chromatographic techniques which are well known to those skilled in the art. The concentration of TFA may range from 5%-100% and other organic solvents may be used such as chloroform. Alternatively, a solution of 3M anhydrous hydrochloric acid in ethyl acetate may be used in place of the TFA-methylene chloride solution with equal efficiency. The ¹H NMR spectra of these acids of Formula 1 indicate that they exist in equilibrium as the closed hemiketal form shown in Formula 1A and that the ratio of Fomula 1 versus Formula 1A is solvent-dependent.

In Scheme II, the synthesis of aryloxy- and arylacyloxymethyl ketones (Formula 2) which possess an N-terminal group other than the Z-group are described. The aspartic acid derivatives of Formula C are the starting material for the synthesis of inhibitors of Formula 2. First the Z-group is removed to generate the N-terminal amine (Formula D) under hydrogenolytic conditions. The reagents and conditions typically used to carry out the hydrogenolytic removal of the Z-group are hydrogen gas, ambient temperature conditions and pressure, 5% palladium on carbon as the catalyst in an alcoholic solvent for example, methanol, optionally containing two equivalents of hydrochloric acid. It is not necessary to purify the intermediate free amine (or the hydrochloride salt if hydrochloric acid is used in the hydrogenolysis), though this material needs to be dry and free of alcohol for the subsequent coupling reaction to proceed in good yield.

The N-terminal amine is then condensed with a carboxylic acid to yield intermediates of Formula E. It is generally necessary to first activate the acid as an acid chloride or mixed anhydride and then react it with the free amine (or hydrochloride salt) in the presence of an organic base, for example, N-methylmorpholine. Alternatively, coupling with acid with the intermediate amine is conducted using amide coupling reagents/conditions employed in peptide coupling chemistry ("The Practice of Peptide Synthesis", M. Bodanszky, Springer-Verlag, NY, 1984; "The Peptides", Vol 1-3, E. Gross and J. Meienhofer, Eds. Academic Press, NY, 1981). Lastly, the t-butyl ester in Formula E is removed with trifluoroacetic acid (as described above) to give the aspartic acid analogs of Formula 2. As in the case of the compounds of Formula 1, the ¹H NMR of components of Formula 2 appear to exist in equilibrium with their corresponding closed hemiketal counterparts of Formula 2A.

The phenols, naphthyls and arylcarboxylic acids used in the reaction with the bromomethyl ketones can be either purchased form commercial sources or synthesized by adopting known procedures. Their synthesis would be readily deduced by those skilled in the art of organic synthesis. By way of example, the preparation of the 2,6-dichloro-3-sulfonamidobenzoic acids are presented in Scheme III. Thus, 2,6-dichlorobenzoic acid (Formula F; available from Aldrich Chemical Co.) is reacted with chlorosulfonic acid to yield the intermediate sulfonyl chloride (Formula G). The electrophilic sulfonyl chloride is reacted with a variety of amines to give the substituted benzoic acids (Formula 3).

Intermediate compounds for use in making the final compounds of the present invention are described in Examples 1-53, 55 and 64 to 70.

### Example 1

### N-Benzyloxycarbonyl-L-aspartic acid bromomethyl ketone β-tert-butyl ester (Formula B)

To a solution of N-benzyloxycarbonyl L-aspartate β-*tert*-butyl ester (Formula A; 10 g, 31 mmol) in 70 ml of anhydrous THF at -15°C was added N-methyl morpholine (4.7 ml, 43.4 mmol) followed by the dropwise addition of ethyl chloroformate (3.9 ml, 40.5 mmol). The reaction mixture was stirred for 30 min at -15°C and the suspension treated with diazomethane in ether (160 ml of a 0.4 in solution in ether, prepared from "DIAZALD®" [Aldrich]) and warmed to room temperature.

The bromomethyl ketone was formed in the same pot by cooling the intermediate diazoketone above followed by the dropwise addition of a 1:1 solution of 48% hydrobromic acid and glacial acetic acid (62 ml). After stirring for 15 min the reaction mixture was poured into a separatory funnel. The aqueous layer was drawn off and discarded. The remaining organic phase was washed with water, saturated aqueous NaHCO₃, brine and dried (MgSO₄). The solvents were removed in vacuo and the title compound so obtained (m.p. 41-43°C) was used in the subsequent displacement reactions without further purification.

### Example 2

### N-Benzyloxycarbonyl-L-Aspartic Acid 2,6-dichlorobenzoyloxymethylketone β-tert-Butyl Ester (Formula C)

The product of Example 1 (0.30 g; 0.76 mM) was dissolved in 12 ml of anhydrous DMF. To this solution was added powdered potassium fluoride (0.11 g; 19 mmol) and 2,6-dichlorobenzoic acid (0.17 g; 0.91 mmol) and the reaction mixture was stirred overnight. The solution was diluted with Et₂O and washed with water, aqueous saturated NaHCO₃, brine and dried (MgSO₄). The ketone so obtained was purified by silica gel chromatography using ethyl acetate/hexane as the eluting solvent (¹H NMR (CDCl₃) δ 7.35 (m, 8H)), 5.90 (d, 2H each), 5.20 (m, 4H), 4.70 (m, 1H), 3.00 and 2.75 (doublet of doublets, 1H each), 1.42 (m, 9H).

In a similar manner, the following compounds of formula C were prepared:

### Example 3

N-Benzyloxycarbonyl-L-aspartic acid 2,6-difluorophenoxymethyl ketone β-*tert*-butyl ester from the product of Example 1 and 2,6-difluorophenol (mp 50-51°C).

### Example 4

N-Benzyloxycarbonyl-L-aspartic acid 2,6-ditrifluoromethyl benzyloxymethyl ketone β-*tert*-butyl ester from the product of Example 1 and 2,6-ditrifluoromethyl benzoic acid (mp 62-63°C).

### Example 5

N-Benzyloxycarbonyl-L-aspartic acid 2,6-dichlorophenoxymethyl ketone β-*tert*-butyl ester from the product of Example 1 and 2,6-dichlorophenol.

### Example 6

N-Benzyloxycarbonyl-L-aspartic acid 2-fluoro-4-(N-morpholinyl sulfonamido)phenoxymethyl ketone β-*tert*-butyl ester from the product of Example 1 and 2-fluoro-4-(N-morpholinylsulfonamido)phenol.

### Example 7

N-Benzyloxycarbonyl-L-aspartic acid 2-chloro-4-(N-thiomorpholinylsulfonamido)phenoxymethyl ketone β-*tert*-butyl ester from the product of Example 1 and 2-chloro-4-(N-thiomorpholinylsulfonamido)phenol.

### Example 8

N-Benzyloxycarbonyl-L-aspartic acid 2,6-dichloro-3-(2-N-morpholinylethoxy)benzoyloxymethyl ketone *β-tert-*butyl ester from the product of Example 1 and 2,6-dichloro-3-(2-N-morpholinylethoxy) benzoic acid.

### Example 9

N-Benzyloxycarbonyl-L-aspartic acid 2,6-dimethoxy benzoyloxymethyl ketone β-*tert*-butyl ester from the product of Example 1 and 2,6-dimethoxybenzoic acid.

### Example 10

N-Benzyloxycarbonyl-L-aspartic acid 2,6-dichloro-3-(benzyloxy) benzoyloxymethyl ketone β-*tert*-butyl ester from the product of Example 1 and 2,6-dichloro-3-(benzoyloxy)benzoic acid.

### Example 11

N-Benzyloxycarbonyl-L-aspartic acid 2-acetamido-6-chlorobenzoyloxymethyl ketone β-*tert*-butyl ester from the product of Example 1 and 2-acetamido-6-chlorobenzoic acid.

### Example 12

N-Benzyloxycarbonyl-L-aspartic acid 2,6-difluorobenzoyloxymethyl ketone β-*tert*-butyl ester from the product of Example 1 and 2,6- difluorobenzoic acid.

### Example 13

N-Benzyloxycarbonyl-L-aspartic acid 3-(N-butylsulfonamido)-2,6-dichlorobenzoyloxymethyl ketone β-*tert*-butyl ester from the product of Example 1 and 3-(N-butylsulfonamido)-2,6-dichlorobenzoic acid.

### Example 14

N-Benzyloxycarbonyl-L-aspartic acid 2,6-dichloro-3-sulfonamido benzoyloxymethyl ketone β-*tert*-butyl ester from the product of Example 1 and 2,6-dichloro-3-sulfonamidobenzoic acid.

### Example 15

N-Benzyloxycarbonyl-L-aspartic acid- 3-(N-benzylsulfonamido)-2,6-dichlorobenzoyloxymethyl ketone β-*tert*-butyl ester from the product of Example 1 and 2,6-dichloro-3-(N-benzylsulfonamido)benzoic acid.

### Example 16

N-Benzyloxycarbonyl-L-aspartic acid 3-(N-[2-aminoacetamidoyl] sulfonamido)-2,6-dichlorobenzoyloxymethyl ketone β-*tert*-butyl ester from the product of Example 1 and 3-(N-[2-aminoacetamidoyl] sulfonamido)-2,6-dichlorobenzoic acid.

### Example 17

N-Benzyloxycarbonyl-L-aspartic acid 2,6-dichloro-3-(N-morpholinylsulfonamido) benzoyloxymethyl ketone β-*tert*-butyl ester from the product of Example 1 and 2,6-dichloro-3-N-morpholinyl sulfonamido)benzoic acid.

### Example 18

N-Methoxycarbonyl-L-Alanine-L-Aspartic Acid 2,6-dichlorobenzoyloxymethyl Ketone β-*tert*-Butyl Ester (Formula E)
Part A: N-benzyloxycarbonyl-L-aspartic acid 2,6-dichlorobenzoyloxymethyl ketone β-*tert*-butyl ester (product of Example 2) (1.02 g, 2 mmol) was dissolved in absolute ethanol (100 ml) containing 6 N aqueous HCI (0.67 ml, 4 mmol). 10% Palladium on carbon (96 mg) was added and the reaction mixture was stirred under an ambient atmosphere of hydrogen gas for approximately 1 hour (thin layer chromotography [5% MeOH-CH₂Cl₂] indicated the disappearance of starting material). The solution was filtered and the solvent was removed in vacuo to give L-aspartic acid 2,6-dichlorobenzoyloxymethyl ketone β-*tert* butyl ester-HCI salt (Formula D) which was used immediately in the subsequent reaction described in Part B.
Part B: A solution of N-methoxycarbonyl-L-alanine (301 mg, 2.05 mmol) in CH₂Cl₂ (10 ml) was cooled to -20°C and isobutylchloroformate (0.28 ml, 2.05 mmol) and N-methylmorpholine (0.23 ml, 2.05 mmol) were added sequentially. The reaction mixture was stirred for 15 minutes and a solution of the product of Part A above added followed by a second addition of N-methyl morpholine (0.23 ml, 2.05 mmol).
   The reaction mixture was stirred for 30 minutes and was then diluted with EtOAc, washed with water, aqueous saturated NaHCO₃, brine and dried (MgSO₄). The solvents were removed in vacuo and the product purified by silica gel chromatography using 40% EtOAc/hexane as eluent to give the desired end-product (0.72 g; 80%).
   In a similar fashion the following compounds of Formula E were prepared:

### Example 19

N-(2-Thienyl)carbonyl-L-aspartic acid 2,6-dichlorobenzoyloxymethyl ketone β-*tert*-butyl ester from the product of Example 2 and 2-thiophene carboxylic acid.

### Example 20

N-Methoxycarbonyl glycine-L-aspartic acid 2,6-dichlorobenzoyloxymethylketone β-*tert*-butyl ester from the product of Example 2 and N-methoxycarbonyl glycine.

### Example 21

N-Methoxycarbonyl-L-phenylalanine-L-aspartic acid 2,6-dichlorobenzoyloxymethyl ketone β-*tert*-butyl ester from the product of Example 2 and N-methoxycarbonyl-L-phenyl alanine.

### Example 22

N-Methoxycarbonyl L-β-(2-thienyl)alanine-L-aspartic acid 2,6-dichlorobenzoyloxymethyl ketone β-*tert*-butyl ester from N-benzyloxycarbonyl-L-aspartic acid 2,6-dichlorobenzoyloxymethyl ketone β-*tert*-butyl ester and N-methoxycarbonyl-L-β-(2-thienyl)alanine.

### Example 23

N-Methoxycarbonyl-L-valine-L-aspartic acid 2,6-dichlorobenzoyloxymethyl ketone β-*tert*-butyl ester from the product of Example 2 and N-methoxycarbonyl-L-valine.

### Example 24

N-Methoxycarbonyl-L-histidine-L-aspartic acid 2,6-dichlorobenzoyloxymethyl ketone β-*tert*-butyl ester from the product of Example 2 and N-methoxycarbonyl-L-histidine.

### Example 25

N-Benzyloxycarbonyl-L-valine-L-aspartic acid 2,6-dichlorobenzoyloxymethyl ketone β-*tert*-butyl ester from the product of Example 2 and N-benzyloxycarbonyl-L-valine.

### Example 26

N-Benzyloxycarbonyl-L-alanine-L-aspartic acid 2,6-dichlorobenzoyloxymethyl ketone β-*tert*-butyl ester from the product of Example 2 and N-benzyloxycarbonyl-L-alanine.

### Example 27

Benzyloxycarbonyl-L-valine-L-alanine-L-aspartic acid 2,6-dichlorobenzoyloxymethyl ketone β-*tert*-butyl ester from the product of Example 2 and N-benzyloxycarbonyl-L-valine-L-alanine.

### Example 28

### N-2-Furoyl-L-Aspartic Acid 2,6-Dichlorobenzoyloxymethylketone β-tert-Butyl Ester (Formula E)

Part A: L-aspartic acid 2,6-dichlorobenzoyloxymethyl ketone β-*tert*-ester-HCl salt (Formula D) was prepared as described in Example 18 Part A and was used immediately in the subsequent reaction described in Part B.
Part B: To a solution of the product of Part A above (2.0 mmol) in CH₂Cl₂ (10 ml) at 0°C was added 2-furoyl chloride (0.21 ml, 2.05 mmol). N-methylmorpholine (0.25 ml; 2.10 mmol) was then added and the reaction mixture stirred for 1 hour as it slowly was allowed to warm to room temperature. The solution was diluted with EtOAc, washed with water, saturated aqueous NaHCO₃, brine and dried (MgSO₄). The solvents were removed in vacuo. The product was purified by silica gel chromatography using 30% EtOAc-hexane as eluent to give the desired end-product (mp 73-74°C).
   In a similar fashion the following compounds of Formula E were prepared:

### Example 29

N-2-Furonylcarbonyl-L-aspartic acid 2,6-dichloro-3-(N-morpholinylsulfonamido) benzoyloxymethyl ketone β-*tert*-butyl ester from the product of Example 17 and 2-furoic acid chloride.

### Example 30

N-(3-Phenylpropionyl)-L-aspartic acid 2,6-dichloro-benzoyloxymethyl ketone β-*tert*-butyl ester from the product of Example 2 and 3-phenylpropionyl chloride.

### Example 31

N-Methoxycarbonyl-L-aspartic acid 2,6-dichlorobenzoyloxymethyl ketone β-*tert*-butyl ester from the product of Example 2 and methyl chloroformate.

### Example 32

N-(N,N-4-Dimethylaminomethyl)benzoyl-L-aspartic acid 2,6-dichlorobenzoyloxymethyl ketone β-*tert*-butyl ester (m.p. 63-65°C) from the product of Example 2 and 4-(N,N-dimethylaminomethyl)benzoyl chloride.

### Example 33

### 3-(N-Butylsulfonamidoyl)-2,6-dichlorobenzoic acid (Formula 3)

Part A: Under an atmosphere of nitrogen gas, a reaction vessel was charged with 2,6-dichlorobenzoic acid (10 g, 53.55 mmol) (Formula F) and chlorosulfonic acid (3 ml, 472 mmol). The reaction mixture was refluxed for 1 hour and cooled to 10°C. The contents of the reaction vessel were poured slowly into 3 l of ice water. The white solid which precipitated was collected by filtration and dried in vacuo (10 mm) at 35°C for 48 hours to give 3-(chlorosulfonyl)-2,6-dichlorobenzoic acid (Formula G) (9.2 g, 61% yield).
Part B: The product from Part A (1.5 g; 5.2 mmol) was dissolved in anhydrous toluene (35 ml) to which was added powdered K₂CO₃ (1.44 g: 10.4 mmol) and n-butylamine (1.0 ml, 10.4 mmol). The reaction mixture was stirred at 25°C for 12 hours. The solution was diluted slowly with 1 M ethereal HCI (20ml) and was then stirred for 30 minutes. The solution was filtered and the resulting filtrate was evaporated to dryness to give crude product. Further purification of the material by silica gel chromatography using EtOAc as the eluent provided the desired end-product. (1.43 g, 85%. ¹H NMR (DMSO) δ 8.11 (t, 1H), 7.98 and 7.71 (doublets, 1H each), 2.75 (m, 2H), 1.55 (m, 2H), 1.32 (m 2H), 0.87 (t, 3H).

In a similar manner, the following compounds of Formula 3 were prepared:

### Example 34

2,6-Dichloro-3-sulfonamidoylbenzoic acid (¹H NMR (DMSO) δ 8.11 (t, 1H), 7.42 and 7.15 (doublets, 1 H each), 7.26 (d, 2H) from the product of Example 33 Part A and 40% aqueous ammonium hydroxide.

### Example 35

3-(N-Benzylsulfonamidoyl)-2,6-dichlorobenzoic acid (¹H NMR (DMSO) δ 8.70 (t, 1H), 7.90 and 7.65 (doublets, 1H each), 7.25 (m, 5H), 4.15 (m, 2H) from the product of Example 33 Part A and benzylamine.

### Example 36

3-(N-[2-Aminoacetamido]sulfonamidoly)-2,6-dichlorobenzoic acid from the product of Example 33 Part A and glycinamide (m. p. 210-213°C.

### Example 37

3-(N-Morpholino)sulfonamidoyl)-2,6-dichlorobenzoic acid from the product of Example 33 Part A and morpholine.

### Example 38

### N-Benzyloxycarbonyl-L-Aspartic Acid 2,6-Dichlorobenzoyloxymethyl Ketone (Formula I)

A solution of β-*tert*-butyl ester of N-benzyloxycarbonyl-L-aspartic acid 2,6-dichlorobenzoyloxymethyl ketone (Example 2) in methylene chloride containing 25% v/v trifluoroacetic acid (20 ml) was stirred for 2 hours at 0°C. The solvent was removed in vacuo and the residue azeotroped three times with methylene chloride to give analytically pure end-product (high resolution mass spectrum for C₂₀H₁₇Cl₂NO₇ found 453.1572).

In a similar fashion, the following compounds of Formulae 1 and 2 were prepared:

### Example 39

N-Benzyloxycarbonyl-L-aspartic acid 2,6-difluorophenoxymethyl ketone (high resolution mass spectrum for C₁₉H₁₇F₂NO₆ found 393.3562) from the β-*tert*-butyl ester of Example 3.

### Example 40

N-Benzyloxycarbonyl-L-aspartic acid 2,6-ditrifluoromethyl benzoyloxymethyl ketone (high resolution mass spectrum for C₂₂H₁₇O₇F₆ found 521.1452) from the β-*tert*-butyl ester of Example 4.

### Example 41

N-Benzyloxycarbonyl-L-aspartic acid 2,6-dichlorophenoxymethylketone (mass spectrum m/z 426 (M+H) from the β-*tert*-butyl ester of Example 5.

### Example 42

N-Benzyloxycarbonyl-L-aspartic acid 2-fluoro-4-(N-morpholinyl sulfonamido)phenoxymethyl ketone (m.p. 65-66°C) from the β-*tert*-butyl ester of Example 6.

### Example 43

N-Benzyloxycarbonyl-L-aspartic acid 2-chloro-4-(N-thiomorpholinylsulfonamido)phenoxymethyl ketone (m.p. 180-181°C) from the β-*tert*-butyl ester of Example 7.

### Example 44

N-Benzyloxycarbonyl-L-aspartic acid 2,6-dichloro-3-(2-N-morpholinylethoxy)benzoyloxymethyl ketone (high resolution mass spectrum for C₂₆H₂₉O₉N₂Cl₂ found 583.1245 ) from the β-*tert*-butyl ester of Example 8.

### Example 45

N-Benzyloxycarbonyl-L-aspartic acid 2,6-dimethoxybenzoyloxy methyl ketone (high resolution mass spectrum for C₂₂H₂₄O₉N found 446.1430 ) from the β-*tert*-butyl ester of Example 9.

### Example 46

N-Benzyloxycarbonyl-L-aspartic acid 2,6-dichloro-3-(benzyloxy) benzoyloxymethyl ketone (high resolution mass spectrum for C₂₇H₂₄O₈NCl₂ found 560.0865 ) from the β-*tert*-butyl ester of Example 10.

### Example 47

N-Benzyloxycarbonyl-L-aspartic acid 2-acetamido-6 -chlorobenzoyloxymethyl ketone (high resolution mass spectrum for C₂₂H₂₂O₈N₂Cl₂ found 477.1044) from the β-*tert*-butyl ester of Example 11.

### Example 48

N-Benzyloxycarbonyl-L-aspartic acid 2,6-difluorobenzoyloxymethyl ketone (high resolution mass spectrum for C₂₀H₁₈O₇NF₂ found 422.1046) from the β-*tert*-butyl ester of Example 12.

### Example 49

N-Benzyloxycarbonyl-L-aspartic acid 3-(N-butylsulfonamido)-2,6-dichlorobenzoyloxymethyl ketone (m.p. 48-50°C) from the β-*tert*-butyl ester of Example 13.

### Example 50

N-Benzyloxycarbonyl-L-aspartic acid 2,6-dichloro-3-sulfonamido benzoyloxymethyl ketone (m.p. 44-46°C) from the β-*tert*-butyl ester of Example 14.

### Example 51

N-Benzyloxycarbonyl-L-aspartic acid 3-(N-benzylsulfonamido)-2,6-dichlorobenzoyloxymethyl ketone (m.p. 66-68°C) from the β-*tert*-butyl ester of Example 15.

### Example 52

N-Benzyloxycarbonyl-L-aspartic acid 3-(N-[2-aminoacetamidoyl] sulfonamido)-2,6-dichlorobenzoyloxymethyl ketone (m.p. 54-56°C) from the β-*tert*-butyl ester of Example 16.

### Example 53

N-Benzyloxycarbonyl-L-aspartic acid 2,6-dichloro-3-(N-morpholinylsulfonamido)benzoyloxymethyl ketone (high resolution mass spectrum for C₂₄H₂₅O₁₀N₂Cl₂ found 603.0594) from the β-*tert*-butyl ester of Example 17.

### Example 54

N-Methoxycarbonyl-L-alanine-L-aspartic acid 2,6-dichlorobenzoyloxymethyl ketone (Anal. calc. for C₁₇H₁₈O₈Cl₂N₂: C, 45.45; H, 4.04; N, 6.24. Found: C, 45.20; H, 4.06; N, 5.98) from the β-*tert*-butyl ester of Example 18.

### Example 55

N-(2-thienyl)carbonyl-L-aspartic acid 2,6-dichlorobenzoyloxymethyl ketone (mass spectrum m/z 430 (M+)) from the β-*tert*-butyl ester of Example 19.

### Example 56

N-Methoxycarbonyl glycine-L-aspartic acid 2,6-dichlorobenzoyloxymethyl ketone (Anal. calc. for C₁₆H₁₆O₈Cl₂N₂: C, 44.16; H, 3.17; N, 6.44. Found: C, 44.24; H, 3.15; N, 6.12) from the β-*tert*-butyl ester of Example 20.

### Example 57

N-Methoxycarbonyl-L-phenylalanine-L-aspartic acid 2,6-dichlorobenzoyloxymethyl ketone (Anal. calc. for C₂₃H₂₂O₈Cl₂N₂: C, 52.59; H, 4.22; N, 5.33. Found: C, 52.98; H, 4.38; N, 5.21) from the β-*tert*-butyl ester of Example 21.

### Example 58

N-Methoxycarbonyl-L-β-(2-thienyl)alanine-L-aspartic acid 2,6-dichlorobenzoyloxymethyl ketone (mass spectrum m/z 531 (M+)) from the β-*tert*-butyl ester of Example 22.

### Example 59

N-Methoxycarbonyl-L-valine-L-aspartic acid 2,6-dichlorobenzoyloxymethyl ketone (m.p. 119-120°C) from the β-*tert*-butyl ester of Example 23.

### Example 60

N-Methoxycarbonyl-L-histidine-L-aspartic acid 2,6-dichlorobenzoyloxymethyl ketone (Anal. calc. for C₂₂H₂₁O₁₀F₃Cl₂N₄: C, 41.99; H, 3.36; N, 8.90. Found: C, 42.08; H, 3.48; N, 8.67; mass spectrum m/z 515 (M+)) from the β-*tert*-butyl ester of Example 24.

### Example 61

N-Benzyloxycarbonyl-L-valine-L-aspartic acid 2,6-dichlorobenzoyloxymethyl ketone (Anal. calc. for C₂₅H₂₆O₈Cl₂N₂: C, 54.26; H, 4.47; N, 5.06. Found: C, 54.06; H, 4.74; N, 4.91) from the β-*tert*-butyl ester of Example 25.

### Example 62

N-Benzyloxycarbonyl-L-alanine-L-aspartic acid 2,6-dichlorobenzoyloxymethyl ketone (mass spectrum m/z 525 (M+)) from the β-*tert*-butyl ester of Example 26.

### Example 63

N-Benzyloxycarbonyl-L-valine-L-alanine-L-aspartic acid 2,6-dichlorobenzoyloxymethyl ketone (Anal. calc. for C₂₈H₃₁O₉Cl₂N₃: C, 53.85; H, 5.00; N, 6.73. Found: C, 54.00; H, 5.04; N, 6.66) from the β-*tert*-butyl ester of Example 27.

### Example 64

N-(2-Furonyl)carbonyl-L-aspartic acid 2,6-dichlorobenzoyloxymethyl ketone (mass spectrum m/z 414 (M+)) from the β-*tert*-butyl ester of Example 28.

### Example 65

N-(2-Furonyl)carbonyl-L-aspartic acid 2,6-dichloro-3-(N-morpholinylsulfonamido)benzoyloxymethyl ketone (mass spectrum m/z 563 (M+)) from the β-*tert*-butyl ester of Example 29.

### Example 66

N-(3-Phenylpropionyl)-L-aspartic acid 2,6-dichlorobenzoyloxymethyl ketone ( ¹H NMR (CDCl₃) δ 7.40 (m, 9H), 5.05 (2xdd, 4H), 4,70 (m, 1H), 2.85 (m, 2H), 2.65 (dd, 1H), 2.60 (dd, 1H), 2.50 (m,2H) from the β-*tert*-butyl ester of Example 30.

### Example 67

N-Methoxycarbonyl-L-aspartic acid 2,6-dichlorobenzoyloxymethyl ketone (1H NMR (DMSO) δ 7.60 (m, 6H), 5.24 (m, 4H), 4.51 (m, 1H), 3.58 (s, 3H), 2.75 (dd, 1H), 2.55 (dd, 1H) from the β-*tert*-butyl ester of Example 31.

### Example 68

N-(4-N,N-dimethylaminomethyl)benzoyl-L-aspartic acid 2,6-dichlorobenzoyloxymethyl ketone (m.p. 55-57°C) from the β-*tert*-butyl ester of Example 32.

### Example 69

N-Benzyloxycarbonyl-D-aspartic acid 2,6-dichlorobenzoyloxymethyl ketone (high resolution mass spectrum for C₂₀H₁₇C₁₂NO₇, found 453.1583) from N-benzyloxycarbonyl-D-aspartic acid bromomethyl ketone β-*tert*-butyl ester and 2,6-dichlorobenzoic acid using the procedures described in Examples 1, 2 and 38.

### Example 70

N-(2-[2,6-dichlorobenzoyloxy])acetyl-L-aspartic acid 2,6-dichlorobenzoyloxymethyl ketone (mass spectrum m/z 551 (M⁺) from N-Benzyloxycarbonyl-L-aspartic acid 2,6-dichlorobenzyloxymethyl ketone β-*tert*-butyl ester and 2-(2,6-dichlorobenzoyloxy)acetic acid using the procedures described in Examples 18 and 38.

### Example 71

N-Benzyloxycarbonyl-L-valine-L-aspartic acid 4-(N,N-diethylsulfonamido)-2,3,5,6-tetrafluorophenoxymethyl ketone (mass spectrum m/z 664 (M+H) from N-benzyloxycarbonyl-L-aspartic acid bromomethyl ketone β-*tert*-butyl ester (Formula B), N-benzyloxycarbonyl-L-valine and 4-(N,N-diethylsulfonamido)-2,3,5,6-tetrafluorophenol using the procedures described in Examples 2, 18 and 38. The 4-(N,N-diethylsulfonamido)-2,3,5,6-tetrafluorophenol was prepared by reacting 2,3,5,6-tetrafluorophenol with chlorosulfonic acid followed by reaction with diethylamine, analogous to the procedure described in Scheme III and Example 33.

Compounds of the present invention were tested for IL-1β protease inhibition activity according to the following protocol:
Partially purified IL-1β protease is stored at -80°C, thawed on ice, and preincubated for 10 minutes at 37°C with 2.5 mM dithiothreitol in a buffer solution containing 10 mM Tris-HCI (pH 8.0) and 25% (v/w) glycerol. Inhibitors are prepared as stock solutions in dimethyl sulfoxide (DMSO). The protease is preincubated with inhibitor in a volume of 20 µl in a 1.5 ml polypropylene microcentrifuge tube for 15 minutes at 37°C. The volume of compound added to the assay is adjusted to yield a DMSO concentration in the preincubation of <15% (v/v). The enzyme assay is then initiated by the addition of substrate (TRITC-AYVHDAPVRS-NH2) to yield a final concentration of 67 µM in a final volume of 30 µl. The reactions are carried out for 60 minutes at 37°C in the dark and are terminated by the addition of 10 µl of 10% trifluoroacetic acid (TFA). Following the addition of 115 µl of 0.1% TFA, the samples are analyzed by high pressure liquid chromatography using a reverse phase (C18) column and elution with an acetonitrile/water/TFA gradient. Substrate and product are monitored by their absorbance at 550 nm and elute at 4.2 and 5.2 minutes, respectively.

**TABLE I**

| Example No. | IC₅₀µm | Name of Compound |
|---|---|---|
| 38 | N-Benzyloxycarbonyl-L-aspartic acid 2,6-dichlorobenzoyloxymethyl ketone | 0.05 |
| 40 | N-Benzyloxycarbonyl-L-aspartic acid 2,6-ditrifluoromethylbenzoyloxymethyl ketone | 0.10 |
| 41 | N-Benzyloxycarbonyl-L-aspartic acid 2,6-dichlorophenoxymethyl ketone | 0.10 |
| 42 | N-Benzyloxycarbonyl-L-aspartic acid 2-fluoro-4-(N-morpholinylsulfonamido)phenoxymethyl ketone | 0.32 |
| 49 | N-Benzyloxycarbonyl-L-aspartic acid 3-(N-butylsulfonamido)-2,6-dichlorobenzoyloxymethylketone | 0.09 |
| 52 | N-Benzyloxycarbonyl-L-aspartic acid 3-(N-[2-aminoacetamidoyl]sulfonamido)-2,6-dichlorobenzoyloxymethyl ketone | 0.06 |
| 53 | N-Benzyloxycarbonyl-L-aspartic acid 2,6-dichloro-3-(N-morpholinylsulfonamido)benzoyloxymethyl ketone | 0.09 |
| 54 | N-Methoxycarbonyl-L-alanine-L-aspartic acid 2,6-dichlorobenzoyloxymethyl ketone | 0.06 |
| 57 | N-Methoxycarbonyl-L-phenylalanine-L-aspartic acid 2,6-dichlorobenzoyloxymethyl ketone | 0.07 |
| 64 | N-(2-furonyl)carbonyl-L-aspartic acid 2, 6-dichlorobenzoyloxymethyl ketone | 0.14 |
| 67 | N-Methoxycarbonyl-L-aspartic acid 2,6-dichlorobenzoyloxymethyl ketone | 0.08 |
| 68 | N-(4-(N,N-dimethylaminomethyl)benzoyl-L-aspartic acid 2, 6-dichlorobenzoyloxymethyl ketone | 0.3 |
| 70 | N-(2-[2,6-dichlorobenzoyloxy])acetyl-L-aspartic acid 2,6-dichlorobenzoyloxymethyl ketone | 0.2 |

## Claims

1. A compound of the formula (I) 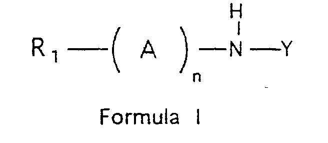 or a pharmaceutically acceptable salt thereof, wherein
n = 1 or 2,
Y= 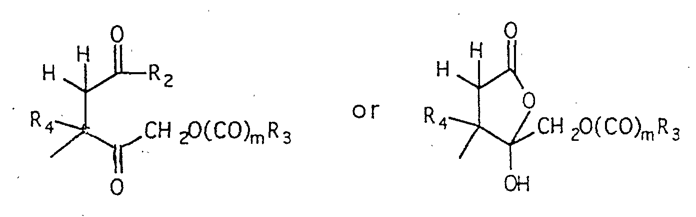
m = 0,1;
R₃ = a singularly or multiply substituted aryl selected from phenyl and naphthyl wherein the substituents are independently selected from the group consisting of:
(1) H;
(2) halogen;
(3) OH
(4) CF₃;
(5) NO₂;
(6) OR₅
(7) COR₉;
(8) NR₆COR₁₀;
(9) CONR₅R₆
(10) SO₂NR₅R₆;
(11) SO₂R₆;
(12) COOR₁₁;
(13) 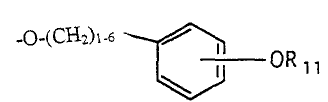 and
(14) lower alkyl and lower cycloalkyl;
R₅ =
(1) lower straight chain or branched alkyl, lower cycloalkyl;
(2) (CR₆R₇)₀₋₆-aryl;
(3) (CR₆R₇)₀₋₆-heteroaryl or
(4) (CR₆R₇)₂₋₆-R₈;
R₆ and R₇ are independently H, lower straight chain or branched alkyl, benzyl, cycloalkyl or aryl wherein aryl is defined as above and heteroaryl includes pyridyl, thienyl, furyl, thiazolyl, imidazolyl, pyrazolyl, isoxazolyl, benzimidazolyl, pyrazinyl, pyrimidyl, quinolyl, isoquinolyl, isothiazolyl, benzofuranyl, isoxazolyl, triazinyl and tetrazolyl;
R₈ =
(1) OCH₂CH₂OR₆;
(2) OCH₂CH₂NR₆R₇
(3) NR₆CH₂CO₂R₆
(4) 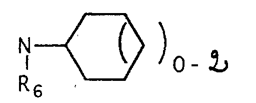
(5) 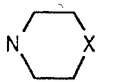 X = O,S and
(6) NR₆R₇ wherein R₆ and R₇ are as above defined;
R₉ =
(1) lower straight chain or branched alkyl, lower cycloalkyl
(2) (CR₆R₇)₀₋₆-aryl;
(3) (CR₆R₇)₀₋₆-heteroaryl; or
(4) (CR₆R₇)₀₋₆-R₈,
wherein R₆, R₇ and R₈ are as above defined;
R₁₀ =
(1) R₉;
(2) OR₁₁; or
(3) NR₆R₁₁, wherein
R₁₁ =
(1) lower straight chain or branched alkyl, lower cycloalkyl
(2) (CR₆R₇)₁₋₆-aryl;
(3) (CR₆R₇)₁₋₆-heteroaryl; or
(4) (CR₆R₇)₂₋₆-R₈,
wherein R₆, R₇, R₈, and R₉ are as above defined;
R₄ = H or deuterium;
R₂ =
(1) OR_{6;}
(2) NR₆OR₇ or
(3) NR₆R₇ wherein
R₆ and R₇ are as above-defined;
A is an aminoacid selected from the group consisting of 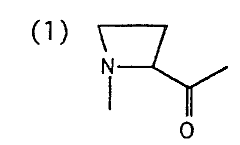 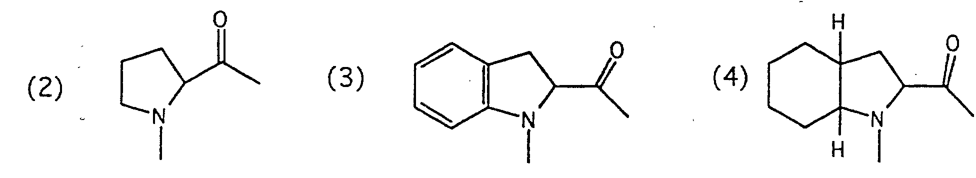 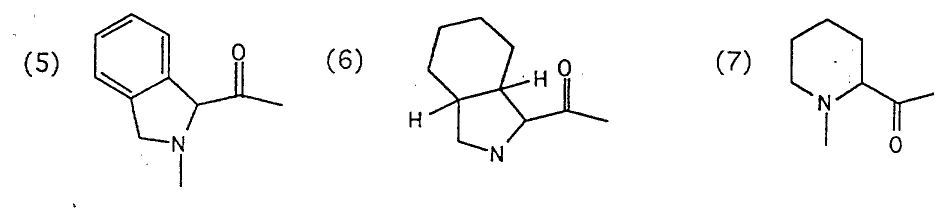 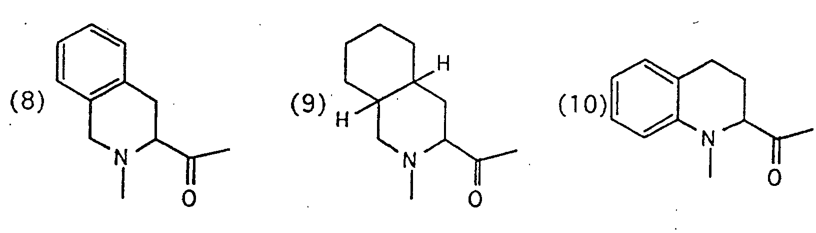 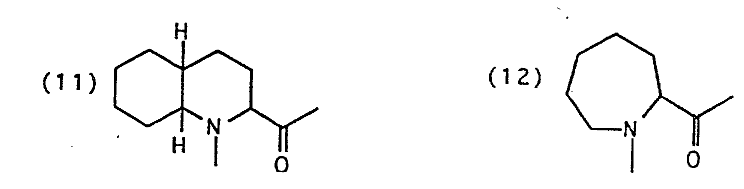 and 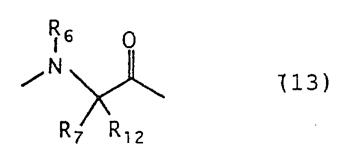 wherein R₆ and R₇ are as defined above; and
R₁₂ is independently
(1) H or
(2) (CR₆R₇)₁₋₆-R₁₃,
wherein R₆ and R₇ are as above-defined;
R₁₃ =
(1) H;
(2) F;
(3) CF₃
(4) OH;
(5) OR₁₁;
(6) NR₆R₁₄
(7) cycloalkyl;
(8) aryl
(9) heteroaryl;
(10) SH
(11) SR_{11;}
(12) CONR₅R₆
(13) COOR₅ or
(14) 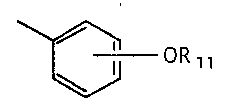 wherein R_{5,} R_{6,} and R₁₁ are as defined above ;
R₁₄ =
(1) R_{7;}
(2) COR_{10;}
(3) SO₂NR₅R₆ or
(4) 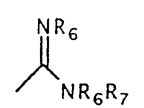 wherein R_{5,} R₆, R₇ and R₁₀ are as defined above;
R₁ is an acyl group of the formula (III) 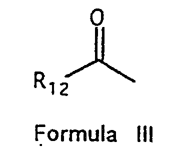 wherein
R₁₂ is
(1) OR5;
(2) NR₅R₆;
(3) R₅;
(4) -CH=CHR₅
(5) 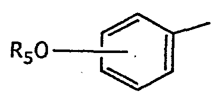
(6) 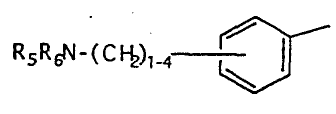
(7) 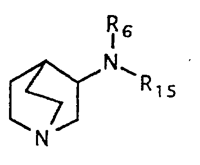 or
(8) 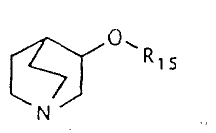 wherein R₁₅ = single bond, (CH₂)₂₋₆-NR₆-, (CH₂)₂₋₆-O- and
R₅ and R₆ are as above defined; or a sulfonyl group of the formula (IV) 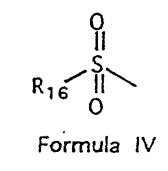 wherein
R₁₆ is
(1) R₅
(2) 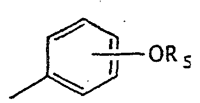 or
(3) 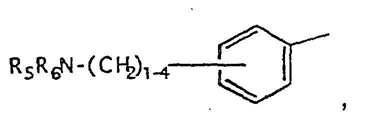 wherein R₅ and R₆ are as above-defined.
the compounds in which all A groups represent a radical of formula (13) being excluded,
with the proviso that when n=1, R₃ does not represent phenyl optionally substituted by one to three radicals selected from lower alkyl, lower alkoxy, trifluoromethyl, halogen, nitro, carbamoyl and (C₁-C₆)alkoxycarbonyl;
it beeing understood that lower alkyl designates an alkyl group having 1 to 7 carbon atoms and lower cycloalkyl designates a cycloalkyl group having 3 to 6 carbon atoms.

2. A pharmaceutical composition comprising a compound of the formula (I) or a pharmaceutically acceptable salt thereof as claimed in claim 1 in combination with a pharmaceutically acceptable carrier.

3. The use of a compound of formula (1) or a pharmaceutically acceptable salt thereof as claimed in claim 1 or a pharmaceutical composition thereof as claimed in claim 2 for the preparation of a medicament for inhibiting interleukin-1β protease activity in a mammal

4. The use of a compound of formula (I) or a pharmaceutically acceptable salt thereof as claimed in claim 1 or a pharmaceutical composition thereof as claimed in claim 2 for the preparation of a medicament for treating IL-1β mediated disease : infectious disease, respiratory disease, inflammatory conditions, immune-based disease, autoimmune disease, bone disease and tumors, in a mammal.

5. A use of a compound of formula (I) or a pharmaceutically acceptable salt thereof as claimed in claim 1 or a pharmaceutical composition thereof as claimed in claim 2 for the preparation of a medicament for treating or preventing a disease or disorder selected from meningitis, salpingitis, septic shock, arthritis, cholangitis, colitis, encephalitis, endocerolitis, hepatitis, pancreatitis, reperfusion injury, hypersensitivity and multiple sclerosis in a mammal.

6. The use according to claim 5, wherein the disease or disorder is arthritis, septic shock, colitis, or pancreatitis.

## Patentansprüche

1. Verbindung der Formel (I) 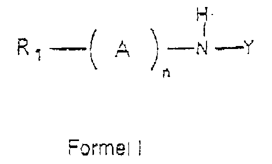 oder ein pharmazeutisch verträgliches Salz davon, wobei
n 1 oder 2 ist,
Y ein Rest der Formel 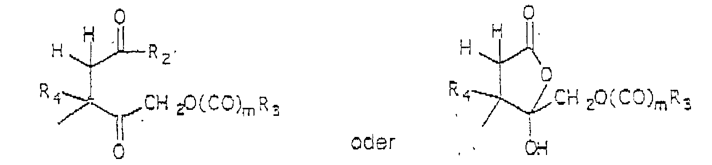 ist,
m 0 oder 1 ist;
R₃ ein einfach oder mehrfach substituierter Arylrest ist, ausgewählt aus einer Phenyl- und Naphtylgruppe, wobei die Substituenten unabhängig voneinander ausgewählt sind aus der Gruppe bestehend aus
(1) einem Wasserstoffatom,
(2) Halogenatom,
(3) einer Hydroxygruppe,
(4) CF₃,
(5) NO₂, oder einem Rest der Formel
(6) OR₅,
(7) COR₈,
(8) NR₆COR₁₀,
(9) CONR₅R₆,
(10) SO₂NR₅R₆,
(11) SO₂R₆,
(12) COOR₁₁,
(13) 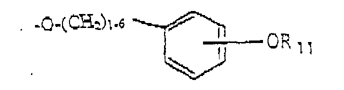 und
(14) einem Niederalkyl- und Niedercycloalkylrest;
R₅
(1) ein gerader oder verzweigter Niederalkyl-, Niedercycloalkylrest, ein Rest der Formel
(2) (CR₆R₇)₀₋₆-Aryl,
(3) (CR₆R₇)₀₋₆-Heteroaryl, oder
(4) (CR₆R₇)₂₋₆-R₈ ist;
R₆ und R₇ unabhängig voneinander ein Wasserstoffatom, ein gerader oder verzweigter Niederalkylrest, eine Benzylgruppe, ein Cycloalkyl- oder Arylrest sind, wobei der Arylrest wie oben definiert ist und der Heteroarylrest eine Pyridyl-, Thienyl, Furyl-, Thiazolyl-, Imidazolyl-, Pyrazolyl-, Isoxazolyl-, Benzimidazolyl-, Pyrazinyl-, Pyrimidyl-, Chinolyl-, Isochinolyl-, Isothiazolyl-, Benzofuranyl-, Isoxazolyl-, Triazinyl- und Tetrazolylgruppe einschließt;
R₈ ein Rest der Formel
(1) OCH₂CH₂OR₆,
(2) OCH₂CH₂NR₆R7,
(3) NF₆CH₂CO₂R₆,
(4) 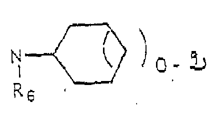
(5) 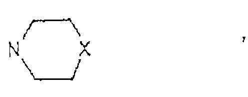 worin X O oder S ist und
(6) NR₆R₇ ist,
wobei R₆ und R₇ wie oben definiert sind;
R₉
(1) ein gerader oder verzweigter Niederalkyl-, Niedercycloalkylrest, ein Rest der Formel
(2) (CR₆R₇)₀₋₆-Aryl,
(3) (CR₆R₇)₀₋₆-Heteroaryl, oder
(4) (CR₆R₇)₀₋₆-R₈ ist,
wobei R₆, R₇ und R₈ wie vorstehend definiert sind;
R₁₀ ein Rest der Formel
(1) R₉,
(2) OR₁₁ oder
(3) NR₆R₁₁ ist,
wobei
R₁₁
(1) ein gerader oder verzweigter Niederalkyl-, Niedercylcoalkyrest, ein Rest der Formel
(2) (CR₆R₇)₁₋₆-Aryl,
(3) (CR₆R₇)₁₋₆-Heteroaryl oder
(4) (CR₆R₇)₂₋₆-R₈ ist, wobei R₆, R₇, R₈ und R₉ wie vorstehend definiert sind;
R₄ ein Wasserstoff- oder ein Deuteriumatom ist;
R₂ ein Rest der Formel
(1) OR₆,
(2) NR₆OR₇ oder
(3) NR₆R₇ ist, wobei R6 und R₇ wie vorstehend definiert sind;
A eine Aminosäure ist, ausgewählt aus der Gruppe bestehend aus 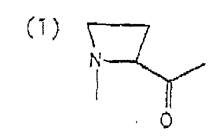 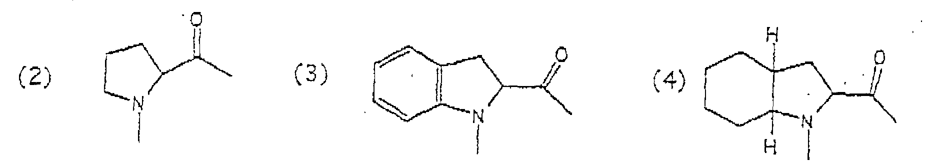 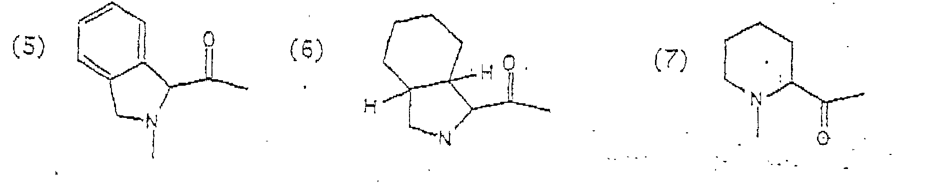 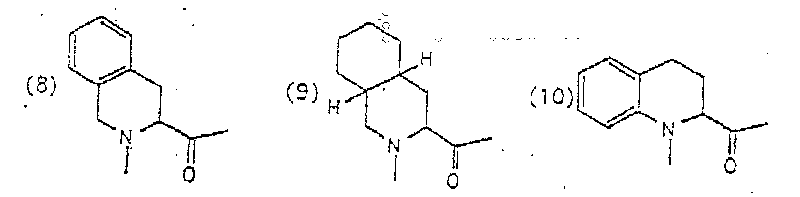 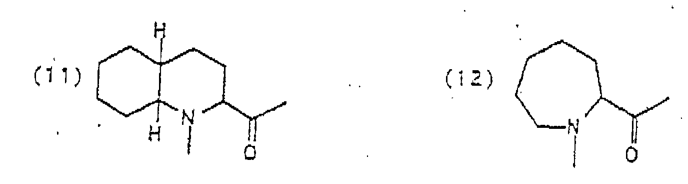 und 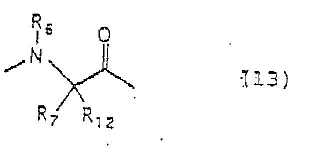 wobei R₆ und R₇ wie vorstehend definiert sind; und
R₁₂ unabhängig
(1) ein Wasserstoffatom oder
(2) ein Rest der Formel (CR₆R₇)₁₋₆-R₁₃ ist, wobei R₆ und R₇ wie vorstehend definiert sind;
R₁₃
(1) ein Wasserstoffatom,
(2) ein Fluoratom,
(3) CF₃,
(4) eine Hydroxygruppe, ein Rest der Formel
(5) OR₁₁,
(6) NR₆R₁₄
(7) ein Cycloalkyl-,
(8) Aryl-,
(9) Heteroarylrest
(10) eine SH-Gruppe, ein Rest der Formel
(11) SR₁₁,
(12) CONR₅R₆,
(13) COOR₅ oder
(14) 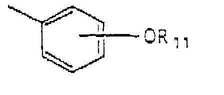 ist, wobei R₅, R₆, und R₁₁ wie vorstehend definiert sind;
R₁₄ ein Rest der Formel
(1) R₇,
(2) COR₁₀,
(3) SO₂NR₅R₆ oder
(4) 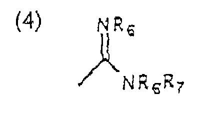 ist, wobei R₅, R₆, R₇ und R₁₀ wie vorstehend definiert sind
R₁ ein Acylrest der Formel (III), 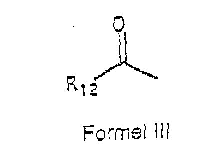 wobei
R₁₂ ein Rest der Formel
(1) OR₅,
(2) NR₅R₆,
(3) R₅,
(4) -CH=CHR₅,
(5) 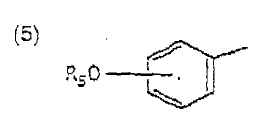
(6) 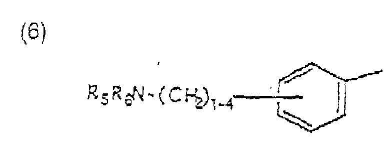
(7) 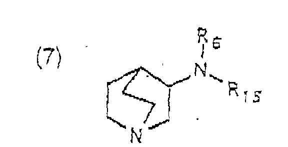 oder
(8) 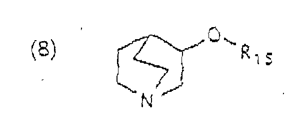 ist,
wobei R₁₅ eine Einfachbindung, ein Rest der Formel (CH₂)₂₋₆-NR₆-, (CH₂)₂₋₆-O- ist und R₅ und R₆ wie vorstehend definiert sind; oder eine Sulfonylgruppe der Formel (IV) ist, 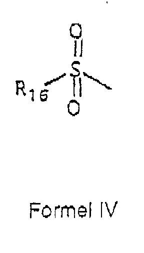 wobei
R₁₆
(1) R₅, ein Rest der Formel
(2) 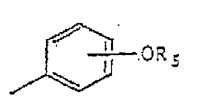 oder
(3) 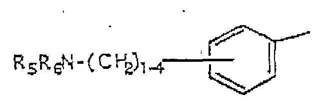 ist, wobei R₅ und R₆ wie vorstehend definiert sind;
wobei die Verbindungen, in welchen alle Reste A einen Rest der Formel (13) darstellen, ausgeschlossen sind,
mit der Maßgabe, daß wenn n 1 ist, R₃ keine Phenylgruppe darstellt, welche gegebenenfalls mit ein bis drei Resten ausgewählt aus einem Niederalkyl-,
Niederalkoxyrest, einer Trifluormethylgruppe, einem Halogenatom, einem Nitro-, Carbamoylgruppe und einem (C₁-C₆)-Alkoxycarbonylrest substituiert ist,
wobei klar ist, daß Niederalkylrest einen Alkylrest, der 1 bis 7 Kohlenstoffatome hat, und Niedercycloalkylrest einen Cycloalkylrest, der 3 bis 6 Kohlenstoffatome hat, bezeichnet.

2. Arzneimittel umfassend eine Verbindung der Formel (1) oder ein pharmazeutisch verträgliches Salz davon nach Anspruch 1 in Kombination mit einem pharmazeutisch verträglichen Träger.

3. Verwendung einer Verbindung der Formel (I) oder eines pharmazeutisch verträglichen Salzes davon nach Anspruch 1 oder ein Arzneimittel davon nach Anspruch 2 für die Herstellung eines Medikaments zur Hemmung der Interleukin-1β-Protease-Aktivität in einem Säuger.

4. Verwendung einer Verbindung der Formel (I) oder eines pharmazeutisch verträglichen Salzes davon nach Anspruch 1 oder eines Arzneimittels davon nach Anspruch 2, für die Herstellung eines Medikaments zur Behandlung einer IL-1β-vermittelten Erkrankung wie: einer Infektionskrankheit, einer Atemwegserkrankung, entzündlichen Zuständen, einer durch das Immunsystem bedingten Erkrankung, einer Autoimmunerkrankung, einer Knochenerkrankung und Tumoren in einem Säuger.

5. Verwendung einer Verbindung der Formel (I) oder eines pharmazeutisch verträglichen Salzes davon nach Anspruch 1 oder eines Arzneimittels davon nach Anspruch 2, für die Herstellung eines Medikaments zur Behandlung oder Vorbeugung einer Erkrankung oder Störung ausgewählt aus Meningitis, Salpingitis, septischem Schock, Arthritis, Cholangitis, Colitis, Encephalitis, Endocerolitis, Hepatitis, Pancreatitis, einer Reperfusionsverletzung, Hypersensibilität und Multipler Sklerose in einem Säuger,

6. Verwendung nach Anspruch 5, wobei die Krankheit oder Störung Arthritis, septischer Schock, Colitis oder Pancreatitis ist.

## Revendications

1. Composé de la formule (I) 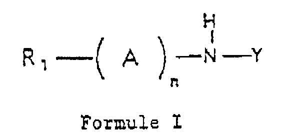 ou son sel pharmaceutiquement acceptable, où
n = 1 ou 2,
Y = 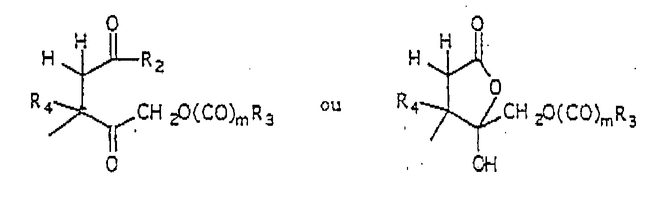
m = 0,1;
R₃ = un aryle singulièrement ou multiplement substitué sélectionné parmi phényle et naphtyle où les substituants sont indépendamment sélectionnés dans le groupe consistant en;
(1) H;
(2) halogène;
(3) OH
(4) CF₃;
(5) NO₂;
(6) OR₅
(7) COR₉;
(8) NR₆COR₁₀;
(9) CONR₅R₆
(10) SO₂NR₅R₆;
(11) SO₂R₆;
(12) COOR₁₁;
(13) 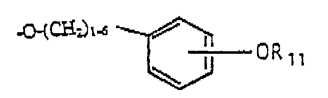 et
(14) alkyle inférieur et cycloalkyle inférieur;
R_{S} =
(1) alkyle inférieur à chaîne droite ou ramifiée, cycloalkyle inférieur;
(2) (CR₆R₇)₀₋₆-aryle;
(3) (CR₆R₇)₀₋₆-hétéroaryle
ou (4) (CR₆R₇)₂₋₆-R₈;
R₆ et R₇ sont indépendamment H, alkyle inférieur à chaîne droite ou ramifiée, benzyle, cycloalkyle ou aryle où aryle est défini comme ci-dessus et hétéroaryle comprend pyridyle, thiényle, furyle, thiazolyle, imidazolyle, pyrazolyle, isoxazolyle, benzimidazolyle, pyrazinyle, pyrimidyle, quinolyle, isoquinolyle, isothiazolyle, benzofuranyle, isoxazolyle, triazinyle et tétrazolyle;
R₈=
(1) OCH₂CH₂OR_{6;}
(2) OCH₂CH₂NR₆R₇
(3) NR₆CH₂CO₂R₆
(4) 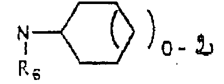
(5) 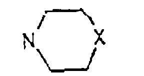 X=O,S et
(6) NR₆R₇
où R₆ et R₇ sont tels que définis ci-dessus;
R₉ =
(1) alkyle inférieur à chaîne droite ou ramifiée, cyclolkyle inférieur
(2) (CR₆R₇)₀₋₆-aryle;
(3) (CR₆R₇)₀₋₆-hétéroaryle; ou
(4) (CR₆R₇)₀₋₆-R₈,
où R₆, R₇ et R₈ sont tels que définis ci-dessus;
R₁₀ =
(1) R₉;
(2) OR₁₁; ou
(3) NR₆R₁₁, où
R₁₁ =
(1) alkyle inférieur à chaîne droite ou ramifiée, cycloalkyle inférieur
(2) (CR₆R₇)1-6-aryle;
(3) (CR₆R₇)₁₋₆-hétéroaryle; ou
(4) (CR₆R₇)₂₋₆-R₈,
où R₆, R₇, R₈ et R₉ sont tels que définis ci-dessus;
R₄ = H ou deutérium;
R₂ =
(1) OR₆;
(2) NR₆OR₇ ou
(3) NR₆R₇
où R₆ et R₇ sont tels que définis ci-dessus;
A est un acide aminé sélectionné dans le groupe consistant en: 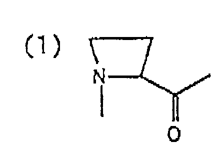 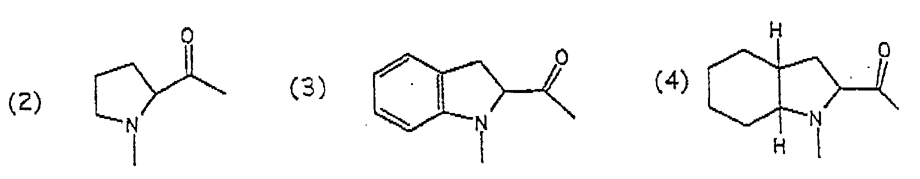 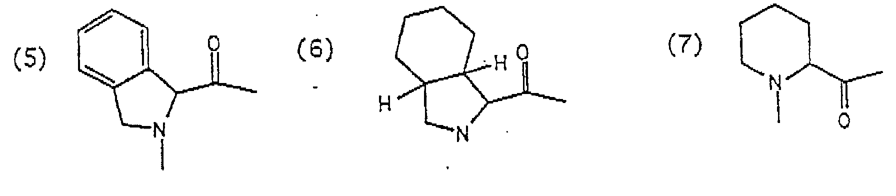 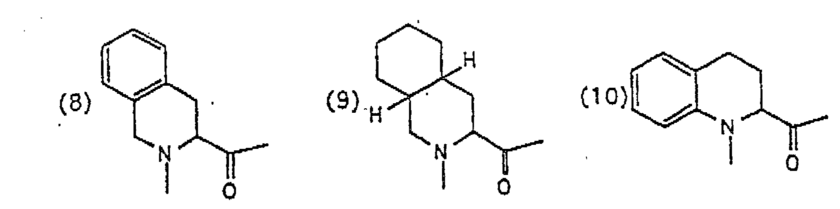 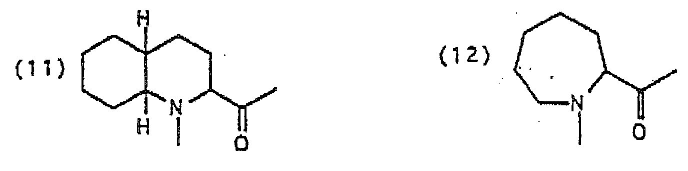 et 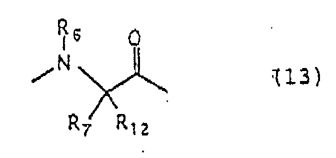 où R₆ et R₇ sont tels que définis ci-dessus; et
R₁₂ est indépendamment
(1) H; ou
(2) (CR₆R₇)₁₋₆-R₁₃,
où R₆ et R₇ sont tels que définis ci-dessus;
R₁₃=
(1) H;
(2) F;
(3) CF₃
(4) OH;
(5) OR₁₁;
(6) NR₆R₁₄
(7) cycloalkyle;
(8) aryle
(9) hétéroaryle;
(10) SH
(11) SR₁₁;
(12) CONR₅R₆
(13) COOR₅ ou
(14) 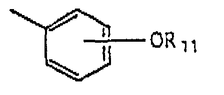
où R₅, R₆, et R₁₁ sont tels que définis ci-dessus;
R₁₄ =
(1) R₇;
(2) COR₁₀;
(3) SO₂NR₅R₆ ou
(4) 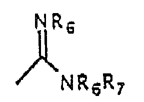 où R₅, R₆, R₇ et R₁₀ sont tels que définis ci-dessus;
R₁ est un groupe acyle de la formule (III) 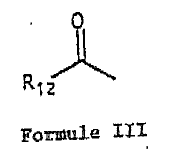 où
R₁₂ est
(1) OR₅;
(2) NR₅R₆;
(3) R₅;
(4) -CH=CHR₅
(5) 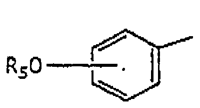
(6) 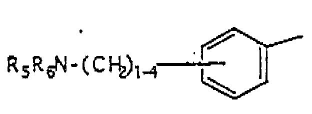
(7) 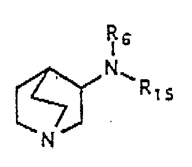 ou
(8) 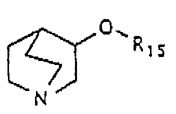
où R₁₅ = simple liaison, (CH₂)₂₋₆-NR₆-, (CH₂)₂₋₆-O-, et
R₅ et R₆ sont tels que définis ci-dessus; ou un groupe sulfonyle de la formule (IV) 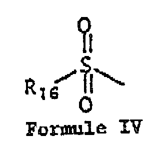 où
R₁₆ est
(1) R₅
(2) 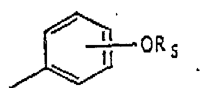 ou
(3) 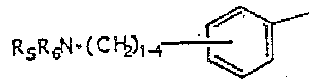
où R₅ et R₆ sont tels que définis ci-dessus.
les composés dans lesquels tous les groupes A représentent un radical de la formule (13) étant exclus,
à condition que quand n=1, R₃ ne représente pas phényle facultativement substitué par un à trois radicaux sélectionnés parmi alkyle inférieur, alcoxy inférieur, trifluorométhyle, halogène, nitro, carbamoyle et alcoxy (C₁-C₆) carbonyle;
en comprenant que alkyle inférieur désigne un groupe alkyle ayant 1 à 7 atomes de carbone et cycloalkyle inférieur désigne un groupe cycloalkyle ayant 3 à 6 atomes de carbone.

2. Composition pharmaceutique comprenant un composé de la formule (I) ou son sel pharmaceutiquement acceptable selon la revendication 1 en combinaison avec un support pharmaceutiquement acceptable.

3. Utilisation d'un composé de formule (I) ou son sel pharmaceutiquement acceptable selon la revendication 1 ou une composition pharmaceutiquement acceptable de celui-ci selon la revendication 2 pour la préparation d'un médicament pour l'inhibition de l'activité de l'interleukine-1β protéase chez un mammifère.

4. Utilisation d'un composé de formule (I) ou son sel pharmaceutiquement acceptable selon la revendication 1 ou sa composition pharmaceutique selon la revendication 2 pour la préparation d'un médicament pour le traitement d'une maladie due à IL-1β; maladie infectieuse, maladie respiratoire, conditions inflammatoires, maladie de base immune, maladie auto-immune, maladie des os et tumeurs, chez un mammifère.

5. Utilisation d'un composé de formule (I) ou son sel pharmaceutiquement acceptable selon la revendication 1 ou sa composition pharmaceutique selon la revendication 2 pour la préparation d'un médicament pour le traitement ou la prévention d'une maladie ou d'un trouble sélectionné parmi méningite, salpingite, choc septique, arthrite, cholangite, colite, encéphalite, endocérolite, hépatite, pancréatite, blessure par reperfusion, hypersensibilité et sclérose multiple chez un mammifère.

6. Utilisation selon la revendication 5, où la maladie ou le trouble est l'arthrite, le choc septique, la colite, ou la pancréatite.
